(19) Europäisches Patentamt
European Patent Office
Office européen des brevets

(11) Veröffentlichungsnummer : **0 324 408 B1**

(12) # EUROPÄISCHE PATENTSCHRIFT

(45) Veröffentlichungstag der Patentschrift : **13.11.91 Patentblatt 91/46**

(51) Int. Cl.[5] : **C07D 211/98**, C07D 265/30, C07D 279/12, C07D 241/04, A61K 31/445, A61K 31/535, A61K 31/495, A61K 31/54

(21) Anmeldenummer : **89100275.0**

(22) Anmeldetag : **09.01.89**

(54) **N-Substituierte-N-Nitroso-aminoacetonitrile, Verfahren zu ihrer Herstellung und ihre Verwendung.**

Die Akte enthält technische Angaben, die nach dem Eingang der Anmeldung eingereicht wurden und die nicht in dieser Patentschrift enthalten sind.

(30) Priorität : **14.01.88 DE 3800830**

(43) Veröffentlichungstag der Anmeldung : **19.07.89 Patentblatt 89/29**

(45) Bekanntmachung des Hinweises auf die Patenterteilung : **13.11.91 Patentblatt 91/46**

(84) Benannte Vertragsstaaten : **AT BE CH DE ES FR GB GR IT LI NL SE**

(56) Entgegenhaltungen :
**DE-A- 1 795 587**
**FR-A- 2 271 818**

(73) Patentinhaber : **CASSELLA Aktiengesellschaft**
**Hanauer Landstrasse 526**
**W-6000 Frankfurt am Main 61 (DE)**

(72) Erfinder : **Schönafinger, Karl, Dr.**
**Holunderweg 8**
**W-8755 Alzenau (DE)**
Erfinder : **Beyerle, Rudi, Dr.**
**An der Pfaffenmauer 44**
**W-6000 Frankfurt am Main 60 (DE)**
Erfinder : **Bohn, Helmut, Dr.**
**Kranzbergring 11**
**W-6369 Schöneck 1 (DE)**

(74) Vertreter : **Urbach, Hans-Georg, Dr. et al**
**Hanauer Landstrasse 526**
**W-6000 Frankfurt am Main 61 (DE)**

**EP 0 324 408 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft pharmakologisch wirksame N-substituierte-N-Nitroso-aminoacetonitrile der allgemeinen Formel I

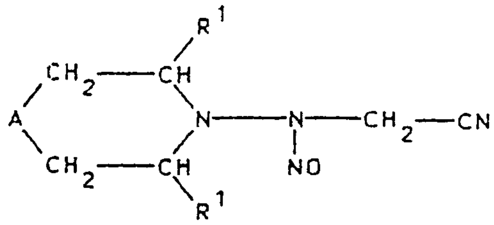

(I)

und ihre pharmakologisch annehmbaren Säureadditionssalze, worin
A den Rest —$CH_2$—, —O—, —$S(O_n)$—, —$N(R^2)$— oder eine direkte Bindung,
$R^1$, $R^2$ Alkyl mit 1 bis 4 C-Atomen oder Phenylalkyl mit 1 bis 4 C-Atomen im Alkylrest,
n die Zahl 0, 1 oder 2,
bedeuten.

Die Erfindung betrifft auch ein Verfahren zur Herstellung der erfindungsgemäßen Verbindungen I und ihre Verwendung.

Alkylreste können geradkettig oder verzweigt sein. Dies gilt auch, wenn sie in Verbindung mit Phenalkyl auftreten.

Für n ist 2 bevorzugt.

Für A sind die direkte Bindung und die zweiwertigen Reste : —$CH_2$—, —O— und —$S(O_2)$— bevorzugt, von denen der Rest —$CH_2$— besonders bevorzugt ist.

Die für $R^1$ und $R^2$ stehenden Alkyl- oder Phenalkylreste können gleich oder verschieden sein. Beispiele für geeignete für $R^1$ und/oder $R^2$ stehende Phenalkylreste sind Benzyl, 2-Phenethyl, 3-Phenyl-propyl, 3-Phenyl-butyl. Beispiele für geeignete für $R^1$ und/oder $R^2$ stehende Alkylreste sind Methyl, Ethyl, n-Propyl, i-Propyl, n-Butyl, i-Butyl, tert.-Butyl. Für $R^1$ und/oder $R^2$ ist Methyl bevorzugt.

Bevorzugte Verbindungen der Formel I sind solche, bei denen A eine direkte Bindung, —O—, —$CH_2$— oder —$S(O_2)$— und $R^1$ Methyl bedeuten.

Eine Verbindung der allgemeinen Formel I kann dadurch hergestellt werden, daß

a) eine Verbindung der allgemeinen Formel II

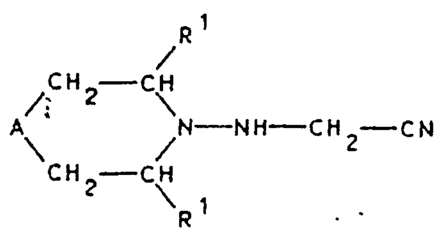

(II)

worin A und $R^1$ die bereits genannten Bedeutungen besitzen, nitrosiert wird, oder daß

b) ein Säureadditionssalz eines substituierten 3-Aminosydnonimins der Formel III

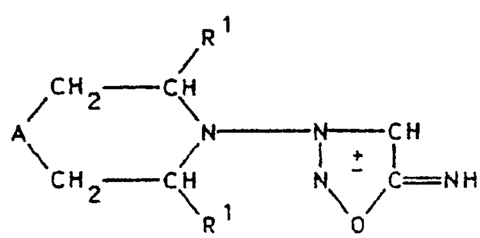

(III)

worin A und $R^1$ die bereits genannten Bedeutungen besitzen, mit einer Base umgesetzt wird und die erhaltene Verbindung für den Fall, daß A = —$N(R^2)$— bedeutet, gegebenenfalls in ein pharmakologisch annehmbares Säureadditionssalz überführt wird.

Die Nitrosierung der Verbindung der Formel II wird in bekannter Weise, zweckmäßigerweise in einem geeigneten inerten Lösungsmittel oder Lösungsmittelgemisch, vorzugsweise in Wasser, normalerweise bei Temperaturen von 0 bis 40°C und vorzugsweise bei Temperaturen von 0 bis 10°C, durchgeführt. Die Nitrosierung erfolgt z.B. mit salpetriger Säure, NO, NOCl oder NO-haltigen Gasgemischen. Zweckmäßigerweise erfolgt die Nitrosierung mit salpetriger Säure, die vorteilhafterweise aus einem Alkalimetallnitrit, z.B. Natriumnitrit, und einer Säure, insbesondere Salzsäure, erzeugt wird. Es ist zweckmäßig, die wäßrige Lösung der Verbindung II mit einer Säure, insbesondere Salzsäure, auf einen pH-Wert von 1 bis 3 einzustellen und das Alkalimetallnitrit in Form einer wäßrigen Lösung zu der gerührten und abgekühlten Lösung der Verbindung zuzutropfen.

Verbindungen der Formel I können auch dadurch hergestellt werden, daß ein Säureadditionssalz einer Verbindung der Formel III, zweckmäßigerweise in wäßriger Lösung, mit einer Base, d.h. einer Verbindung, die in Wasser eine alkalische Reaktion einstellt, wie z.B. einem Alkalihydroxid, wie z.B. Lithium-, Natrium- oder Kaliumhydroxid, einem Alkalikarbonat, wie z.B. Lithium-, Kalium- oder Natriumkarbonat oder einem Alkalibikarbonat, wie z.B. Natriumbikarbonat, oder einem Amin, insbesondere einem tertiären Amin, wie z.B. Triethylamin, behandelt wird. Die Umsetzung wird normalerweise bei 10 bis 40°C, vorzugsweise bei Raumtemperatur, durchgeführt. Es wird mindestens soviel Base zugesetzt, daß der Säurerest vollständig gebunden wird. In der Regel wird das Säureadditionssalz in Wasser oder einem Gemisch aus Wasser und einem Lösungsmittel gelöst und so viel Base zugesetzt, bis die wäßrige Lösung alkalisch reagiert. Die Bindung des Säurerestes kann auch mit einem Austauscherharz erfolgen.

Die benötigten Ausgangsverbindungen der allgemeinen Formel II können in an sich bekannter Weise nach der Strecker'schen Aminonitrilsynthese aus Verbindungen der allgemeinen Formel IV

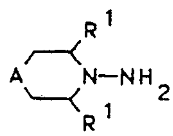

(IV)

worin A und $R^1$ die bereits genannten Bedeutungen besitzen, durch Umsetzung mit Formaldehyd und Blausäure bzw. Natriumcyanid in einem geeigneten Lösungsmittel, z.B. Wasser, hergestellt werden, wobei eine Verbindung der allgemeinen Formel II entsteht, die durch Nitrosierung in die Verbindung I überführt wird. Aus der Lösung der dabei erhaltenen Verbindung I kann die Verbindung I isoliert werden.

Ausgangsverbindungen der Formel III können dadurch hergestellt werden, daß eine Verbindung der Formel I oder die bei der Nitrosierung der Verbindung II anfallende Lösung cyclisiert wird. Die Cyclisierung der Verbindung I wird in einem geeigneten organischen oder anorganischen Lösungs-, Dispergier- oder Verdünnungsmittel unter Zusatz eines Cyclisierungsmittels, normalerweise bei Temperaturen von −10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert von 3 oder darunter einstellen, also z.B. starke Säuren, wie Mineralsäuren, wie Schwefel-, Salpeter- oder Phosphorsäure, vorzugsweise Chlorwasserstoff, aber auch starke organische Säuren, wie Sulfonsäuren oder Trifluoressigsäure. Die Cyclisierung wird normalerweise unter Eiskühlung durchgeführt. Von dem Cyclisierungsmittel kommt z.B. bezogen auf 1 mol der Verbindung der Formel I 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird direkt ein Säureadditionssalz der Verbindung III erhalten.

Geeignete Lösungs-, Dispergier- oder Verdünnungsmittel sind z.B. : Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol ; Ether, insbesondere solche mit 2 bis 8 C-Atomen im Molekül, wie z.B. Diethylether, Methylethylether, Di-n-propyl-ether, Di-isopropyl-ether, Methyl-n-butylether, Methyl-tert-butylether, Ethyl-propylether, Di-butyl-ether, Tetrahydrofuran, 1,4-Dioxan, 1,2-Dimethoxyethan, Bis-β-methoxyethylether ; Oligoethylen-glykol-dimethyl-ether, wie z.B. Tetraglyme oder pentaglyme ; Carbonsäurealkylester, insbesondere solche mit 2 bis 10 C-Atomen im Molekül, wie z.B. Ameisensäure-methyl-, -ethyl-, -butyl- oder -isobutylester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester ; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon ; aliphatische

Kohlenwasserstoffe, wie z.B. Hexan, Heptan, niedrig-und hochsiedende Petrolether, Spezialbenzine und Testbenzin ; cycloaliphatische Kohlenwasserstoffe, wie z.B. Cyclopentan, Cyclohexan, Methylcyclohexan, Tetralin, Decalin ; aromatische Kohlenwasserstoffe, wie z.B. Benzol, Toluol, o-, m- und p-Xylol, Ethylbenzol ; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol ; Hexamethylphosphorsäuretriamid ; Sulfoxide, wie z.B. Dimethyl-sulfoxid ; Tetramethylensulfon ; Wasser. Auch Gemische verschiedener Lösungs- oder Dispergiermittel können verwendet werden, beispielsweise Wasser-Methanol oder vorzugsweise Essigsäureethylester-Methanol.

Die bei der Nitrosierung der Verbindung II anfallende Lösung der Verbindung I kann direkt der Cyclisierungsreaktion unterworfen werden. Normalerweise ist es jedoch für die anschließende Cyclisierung angebracht, die Nitrosoverbindung I zunächst in einem geeigneten organischen Lösungsmittel aufzunehmen und in ihm, gegebenenfalls nach Zusatz eines weiteren Lösungsmittels, die Cyclisierung zu der Verbindung der Formel III durchzuführen. Es kann für die Herstellung einer Verbindung der Formel I zweckmäßig sein, aus der bei der Nitrosierung der Verbindung II an fallenden Lösung der Verbindung I die Verbindung I nicht direkt zu isolieren, sondern zu einer Verbindung der Formel III zu cyclisieren und hieraus die Verbindung der Formel I zu gewinnen.

Die erfindungsgemäßen Verbindungen der Formel I können in verschiedenen Konfigurationen in cis- oder in trans-Form vorliegen, wobei die trans-Formen als racemische Gemische oder in optisch aktiver Form vorliegen können. Für die Herstellung dieser verschiedenen Isomeren können an sich bekannte Verfahren, wie selektive Synthese, chirale Synthese oder Trennung von racemischen Gemischen nach bekannten Methoden Anwendung finden.

Die Verbindungen der allgemeinen Formel I können, für den Fall, daß A = —N($R^2$)— bedeutet, mit anorganischen oder organischen Säuren Säureadditionssalze bilden. Zur Bildung derartiger Säureadditionssalze sind anorganische oder organische Säuren geeignet. Geeignete Säuren sind beispielsweise Chlorwasserstoff, Bromwasserstoff, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen-, Adipinsäure oder Naphthalindisulfonsäuren, insbesondere Naphthalindisulfonsäure (1, 5). Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Die Verbindungen der allgemeinen Formel IV sind zum Teil bekannt (vergl. z.B. C.G. Overberger et al, J. Amer. Chem. Soc. 77, (1955) 4100) bzw. lassen sich, ausgehend von Verbindungen der allgemeinen Formel V

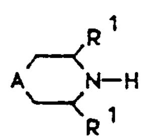

(V)

dadurch herstellen, daß entweder

a) eine Verbindung der Formel V zur N-Nitrosoverbindung VI nitrosiert und anschließend, zweckmäßigerweise mit Lithiumaluminiumhydrid, reduziert wird :

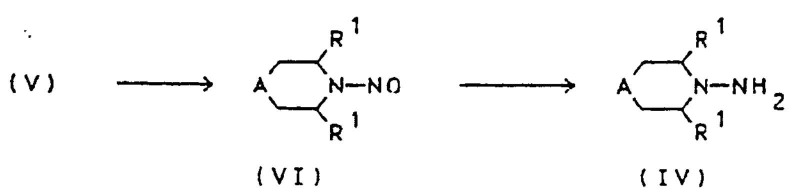

oder daß in an sich bekannter Weise

b) eine Verbindung der Formel V mit Kaliumcyanat im sauren Medium in das Harnstoffderivat VII überführt wird, das dann durch Oxydation mit Natriumhypochlorit nach dem Hoffmann-Abbau in die Verbindung IV überführt wird :

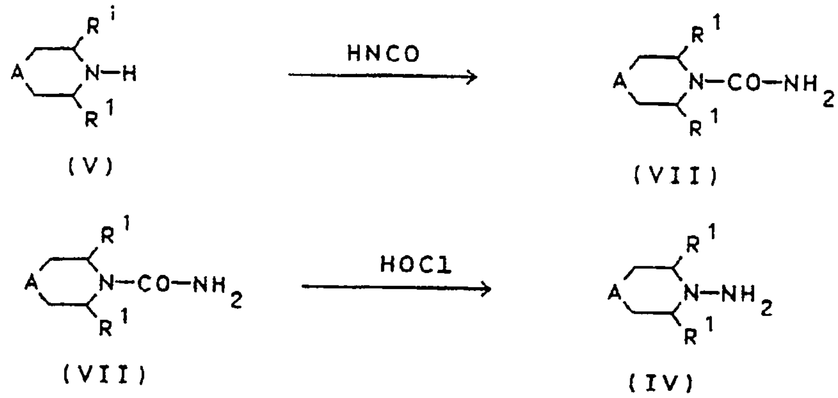


(V) (VII)

(VII) (IV)

Ausgangsverbindungen der Formel V sind bekannt (vergl. z.B. Backer, van der Ley, Rec. Trav. Chim. Pays-Bas 70, (1951) 564 ; Berlin, Sytschewa, Z. obsc. Chim. 20, (1950) 640 ; Idson, Spoerri, J. Amer. Chem. Soc. 76, (1954) 2902) bzw. lassen sich nach den für diese Verbindungsklassen bekannten Verfahren herstellen. So lassen sich Verbindungen der Formel V z.B. aus Verbindungen der allgemeinen Formel VIII

$$R^{1'}=CH-CH_2-A-CH_2-CH=R^{1'} \quad (VIII)$$

worin A —SO— oder —$S(O_2)$— und

$R^{1'}$ einen Rest bedeutet, der bei der Umsetzung mit Ammoniak durch Addition von Wasserstoff in den bereits genannten Rest $R^1$ übergeht und die nach an sich bekannten Methoden darstellbar sind, durch Ringschluß mit Ammoniak herstellen. Die Umsetzung mit Ammoniak kann bei Temperaturen von 20 bis 150°C, vorzugsweise bei 60 bis 100°C, mit oder ohne Lösungsmittel durchgeführt werden.

Führt man die Umsetzung der Verbindungen der Formel VIII nicht mit Ammoniak sondern unter den vorgenannten Bedingungen mit Hydrazin durch, so kann man bei dem Ringschluß direkt zu Verbindungen der Formel IV gelangen.

Die Verbindung der allgemeinen Formel I und ihre gegebenenfalls existierenden pharmakologisch annehmbaren Säureadditionsalze besitzen wertvolle pharmakologische Eigenschaften. Besonders ausgeprägt ist ihre Wirkung auf das Herz-Kreislauf-System. Verglichen mit bekannten, in 3-Stellung substituierten Sydnoniminverbindungen, z.B. solchen der EP-B-59356, sowie der im Handel befindlichen Verbindung Molsidomin, besitzen sie z.B. überraschenderweise eine wesentlich längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die Verbindungen der Formel I und ihre gegebenenfalls existierenden pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen nen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler

oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise : β-Rezeptorenblocker, wie z.B. Propranolol, Pindolol, Metoprolol ; Vasodilatatoren, wie z.B. Carbochromen ; Beruhigungsmittel, wie z.B. Barbitursäurederivate, 1,4-Benzodiazepine und Meprobamat ; Diuretica, wie z.B. Chlorothiazid ; das Herz tonisierende Mittel, wie z.B. Digitalispräparate ; blutdrucksenkende Mittel, wie z.B. Hydralazin, Dihydralazin, Prazosin, Clonidin, Rauwolfia-Alkaloide ; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Zum Nachweis der antianginösen Wirkung der erfindungsgemäßen Verbindungen wurden Untersuchungen an Bastardhunden beiderlei Geschlechts in Pentobarbital-Narkose (30 bis 40 mg/kg i.v.) oder in Urethan-Chloralose-Narkose (3 ml/kg Urethan-Chloralose-Gemisch i.v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit einem Ultrarotabsorptionsschreiber) betrug zwischen 4,5 und 5 Vol.%. Während des gesamten Versuchs erhielten die Tiere mit Pentobarbital-Narkose eine Dauerinfusion von Pentobarbital i.v. = 4 mg (in 6 ml)/kg/h, um eine konstante Narkosetiefe zu gewährleisten. Die Tiere mit Urethan-Chloralose-Narkose erhielten keine Dauerinfusion. Die Infusion wurde durch die Vena cephalica gegeben. Nach der Präparation des Versuchstieres wurde ca. 1 Stunde gewartet, bis sich alle haemodynamischen Parameter eingestellt hatten (steady state). Danach wurde mit dem eigentlichen Versuch begonnen.

Zur Bestimmung des mittleren peripheren Blutdrucks (= BD) wurden der systolische und diastolische Blutdruck peripher in der Arteria femoralis über einen Statham-Druck-aufnehmer gemessen. Ein über die Arteria carotis in den linken Ventrikel geschobener Millar-Tip-Katheter lieferte das Signal für den linksventrikulären enddiastolischen Druck (= LVEDP) und die Herzfrequenz (= HF).

Die erhaltenen Ergebnisse sind in der nachfolgenden Tabelle angegeben.

| Substanz | Dosis mg/kg | BD Δmm Hg | LVEDP Δmm Hg | HF Δ b/min | WD min |
|---|---|---|---|---|---|
| A | 0,3 | -35 | -3 | +6 | 150 |
| Mol | 0,1 | -18 | -2,3 | +3 | 90 |

In der vorstehenden Tabelle bedeuten :

A = N-(2,6-Dimethylpiperidin-1-yl)-N-nitroso-aminoacetonitril
Mol = Vergleichssubstanz Molsidomin (3-(Morpholin-1-yl)-N-ethoxycarbonyl-sydnonimin)
BD = Mittlerer peripherer Blutdruck
LVEDP = Linksventrikulärer enddiastolischer Druck
HF = Herzfrequenz (b/min=Schläge pro Minute)
WD = Wirkungsdauer

Aus der DE-A-1795587 ist aus Beispiel 5 das N-Nitroso-N-α-pipecolinoaminoacetonitril (= N-(2-Methylpiperidin-1-yl)-N-nitrosoaminoacetonitril) bekannt. Die Verbindungen der DE-A-1795587 werden als Heilmittel in

der Therapie der Hypertension, der Raynaud'schen Krankheit und bei Herzinsuffizienz bezeichnet.

In den nachfolgenden Beispielen sind, sofern nichts Anderes angegeben ist, Prozente in Gewichtsprozenten angegeben. Angegebene Verhältnisse zwischen Komponenten von Lösungs- oder Fließmitteln sind Volumenverhältnisse. Die Angabe "Zers." bedeutet Zersetzung.

Beispiel 1

3-(3,5-Dimethyl-thiomorpholin-1,1-dioxid-4-yl)-N-nitroso-aminoacetonitril

a) Die Mischung aus 32,2 g Diallylsulfon, 55 g Hydrazinhydrat und 54 ml Wasser wird 30 Minuten zum Sieden erhitzt. Der Ansatz wird im Eisbad gerührt, der Niederschlag abgesaugt und aus Ethanol umkristallisiert.
Ausbeute : 16,2 g 4-Amino-3,5-dimethyl-thiomorpholin-dioxid
Schmelzpunkt : 181 bis 183°C

b) Die Mischung aus 16,2 g 4-Amino-3,5-dimethyl-thiomorpholin-dioxid, 9,1 g konz. Salzsäure, 80 ml Wasser und 5,6 g Natriumcyanid wird auf 5°C abgekühlt und mit 9,1 g einer 39%igen Formaldehydlösung versetzt (pH = 7 bis 7,5). Die Reaktionsmischung wird dann 4 Stunden bei Raumtemperatur gerührt. Unter Eiskühlung wird die Mischung sauer (pH = 1) gestellt und eine Lösung von 6,3 g Natriumnitrit in 25 ml Wasser zugetropft. Die Mischung wird bei Raumtemperatur noch 3 Stunden nachgerührt und der Feststoff abgesaugt.
Ausbeute : 16,0 g N-(3,5-Dimethyl-thiomorpholin-1,1-dioxid-4-yl)-N-nitroso-aminoacetonitril
Schmelzpunkt : 163 bis 165°C

Beispiel 2

N-(2,6-Dimethylpiperidin-1-yl)-N-nitroso-aminoacetonitril

Die wäßrige Lösung von 11,5 g 3-(2,6-Dimethylpiperidin-1-yl)-sydnonimin-hydrochlorid wird mit 6 g $NaHCO_3$ versetzt und 2 Stunden gerührt. Das Produkt wird mit Essigester ausgeschüttelt, die organische Phase abgetrennt, getrocknet und eingeengt. Der Rückstand wird mit Petrolether verrührt und abgesaugt.
Ausbeute : 6,5 g Schmelzpunkt : 90-91°C

Das benötigte Ausgangsprodukt 3-(2,6-Dimethylpiperidin-1-yl)-sydnonimin-hydrochlorid wird wie folgt hergestellt :

a) Zur eisgekühlten Mischung von 30,0 g 1-Amino-2,6-dimethylpiperidin, 20 g konz. Salzsäure und 100 ml Wasser wird die Lösung von 11,5 g Natriumcyanid in 20 ml Wasser gegeben und der pH-Wert mit Salzsäure auf 6,5 eingestellt. Dann werden 19,3 g einer 39%igen wäßrigen Formaldehyd-Lösung zugesetzt und die Reaktionsmischung 3 Stunden bei 0°C und weitere 3 Stunden bei Raumtemperatur gerührt. Das Produkt wird mit Essigsäure-ethylester extrahiert, die Essigsäure-ethylesterphase mit verdünntem Eisessig gewaschen und über Natriumsulfat getrocknet. Nach dem Einengen verbleibt ein öliger Rückstand von 29,1 g 3-(2,6-Dimethylpiperidin-1-yl)-aminoacetonitril, der ohne weitere Reinigung wie folgt weiterverarbeitet wird :

b) Zur Mischung von 29,1 g N-(2,6-Dimethylpiperidin-1-yl)-aminoacetonitril, 100 ml Wasser, 100 ml Essigsäureethylester und 12 g konz. Salzsäure wird die Lösung von 13,4 g Natriumnitrit in 10 ml Wasser getropft und nach 5 Stunden Rühren bei Raumtemperatur die organische Phase abgetrennt, mit 20 ml Methanol verdünnt und im Eisbad gerührt. Nach dem Einleiten von Salzsäure bis zur Sättigung wird 15 Stunden bei Raumtemperatur nachgerührt und im Wasserstahl-Vakuum eingeengt. Der Rückstand kristallisiert biem Verrühren mit Diethylether. Er wird abgesaugt und aus Acetonitril umkristallisiert.
Ausbeute : 18,2 g 3-(2,6-Dimethylpiperidin-1-yl)-sydnonimin-hydrochlorid
Schmelzpunkt : 136-137°C (Zers.)

In den nachfolgenden Beispielen A bis F werden pharmazeutische Präparate beschrieben.

Beispiel A

Gelatineweichkapseln, enthaltend 5 mg Wirkstoff pro Kapsel :

| | | pro Kapsel |
|---|---|---|
| Wirkstoff | | 5 mg |
| Aus Kokosfett fraktioniertes Triglycerid-Gemisch | | 150 mg |
| Kapselinhalt | | 155 mg |

Beispiel B

Injektionslösung, enthaltend 1 mg Wirkstoff pro ml :

| | | pro ml |
|---|---|---|
| Wirkstoff | | 1,0 mg |
| Polyethylenglycol 400 | | 0,3 ml |
| Natriumchlorid | | 2,7 mg |
| Wasser zu Injektionszwecken | ad | 1 ml |

Beispiel C

Emulsion, enthaltend 3 mg Wirkstoff pro 5 ml

| | | pro 100 ml Emulsion |
|---|---|---|
| Wirkstoff | | 0,06 g |
| Neutralöl | | q.s. |
| Natriumcarboxymethylcellulose | | 0,6 g |
| Polyoxyethylen-stearat | | q.s. |
| Glycerin rein | | 0,2 bis 2,0 g |
| Geschmacksstoff | | q.s. |
| Wasser (entsalzt oder destilliert) | ad | 100 ml |

Beispiel D

Rektale Arzneiform, enthaltend 4 mg Wirkstoff pro Suppositorium

| | | pro Suppositorium |
|---|---|---|
| Wirkstoff | | 4 mg |
| Suppositoriengrundmasse | ad | 2 g |

Beispiel E

Tabletten, enthaltend 2 mg Wirkstoff pro Tablette

| | pro Tablette |
|---|---|
| Wirkstoff | 2 mg |
| Maisstärke | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboxymethylstärke | 25 mg |
| | 309 mg |

Beispiel F

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

## Patentansprüche

**Patentansprüche für folgenden Vertragsstaaten : AT, BE, CH, DE, FR, GB, IT, LI, NL, SE**

1. N-Substituierte-N-Nitroso-aminoacetonitrile der allgemeinen Formel I

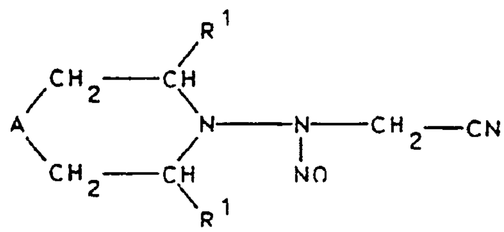

(I)

und ihre pharmakologisch annehmbaren Säureadditionssalze, worin
A den Rest $-CH_2-$, $-O-$, $-S(O_n)-$, $-N(R^2)-$ oder eine direkte Bindung,
$R^1$, $R^2$ Alkyl mit 1 bis 4 C-Atomen oder Phenylalkyl mit 1 bis 4 C-Atomen im Alkylrest,
n die Zahl 0, 1 oder 2,
bedeuten.

2. N-Substituierte-N-Nitroso-aminoacetonitrile nach Anspruch 1, dadurch gekennzeichnet, daß $R^1$ Methyl bedeutet.

3. N-Substituierte-N-Nitroso-aminoacetonitrile nach Anspruch 1 und/oder 2, dadurch gekennzeichnet, daß A $-CH_2-$, eine direkte Bindung, $-S(O_2)-$ oder $-O-$ bedeutet.

4. N-(2,6-Dimethylpiperidin-1-yl)-N-nitroso-aminoacetonitril.

5. Verfahren zur Herstellung der in einem oder mehreren der Ansprüche 1 bis 3 angegebenen Verbindungen der Formel I oder einer der in dem Ansprüchen 4 angegebenen Verbindungen, dadurch gekennzeichnet, daß

a) eine Verbindung der allgemeinen Formel II

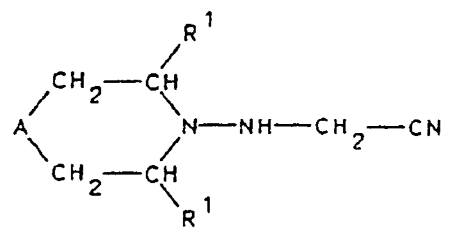

(II)

worin A und $R^1$ die in Anspruch 1 genannten Bedeutungen besitzen, nitrosiert wird, oder daß

b) ein Säurealditionssalz eines substituierten 3-Aminosydnonimins der Formel III

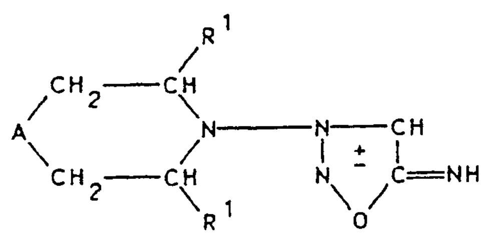

(III)

worin A und $R^1$ die in Anspruch 1 genannten Bedeutungen besitzen, mit einer Base umgesetzt wird und die erhaltene Verbindung für den Fall, daß A = —N($R^2$)— bedeutet, gegebenenfalls in ein pharmakologisch annehmbares Säureadditionssalz überführt wird.

6. N-Substituierte-N-Nitroso-amino-acetonitrile eines oder mehrerer der Ansprüche 1 bis 4 und/oder ihre gegebenenfalls existierenden pharmakologisch annehmbaren Säureadditionssalze als pharmakologische Wirkstoffe zur Anwendung bei der Bekämpfung und Vorbeugung cardiovaskulärer Erkrankungen.

**Patentansprüche für folgenden Vertragsstaaten : ES, GR**

1. Verfahren zur Herstellung von N-substituierten N-Nitroso-aminoacetonitrilen der allgemeinen Formel I

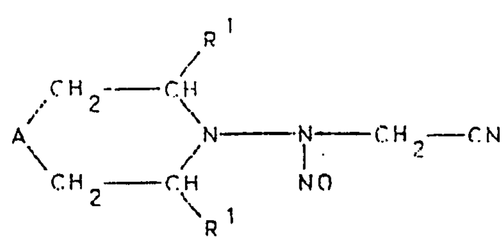

(I)

und ihrer pharmakologisch annehmbaren Säureadditionssalze, worin

A den Rest —$CH_2$—, —O—, —S($O_n$)—, —N($R^2$)— oder eine direkte Bindung,

$R^1$, $R^2$ Alkyl mit 1 bis 4 C-Atomen oder Phylalkyl mit 1 bis 4 C-Atomen im Alkylrest,

n die Zahl 0, 1 oder 2,

bedeuten, dadurch gekennzeichnet, daß

a) eine Verbindung der allgemeinen Formel II

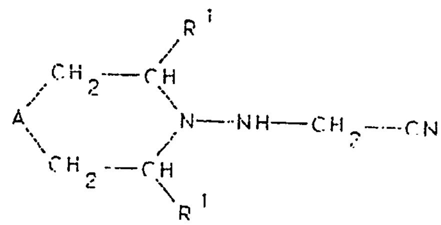

(II)

worin A und $R^1$ die genannten Bedeutungen besitzen, nitrosiert wird, oder daß

b) ein Säureadditionssalz eines substituierten 3-Amino-sydnonimins der Formel III

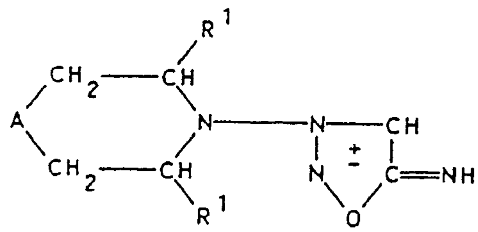

(III)

worin A und $R^1$ die genannten Bedeutungen besitzen, mit einer Base umgesetzt wird und die erhaltene Verbindung für den Fall, daß A = —N($R^2$)— bedeutet, gegebenenfalls in ein pharmakologisch annehmbares Säureadditionssalz überführt wird.

2. Verfahren nach Anspruch 1, dadurch gekennzeichnet, daß eine Verbindung der Formel II oder III mit $R^1$ = Methyl eingesetzt wird.

3. Verfahren nach Anspruch 1 und/oder 2, dadurch gekennzeichnet, daß eine Verbindung der Formel II und/oder III mit A = —$CH_2$—, —S($O_2$)—, —O— oder einer direkten Bindung eingesetzt wird.

4. Verfahren nach einem oder mehreren der Ansprüche 1 bis 3, dadurch gekennzeichnet, daß eine Verbindung der Formel II oder III mit A = —$CH_2$— und $R^1$ = Methyl eingesetzt wird.

5. Verwendung N-substituierter N-Nitroso-aminoacetonitrile der allgemeinen Formel I

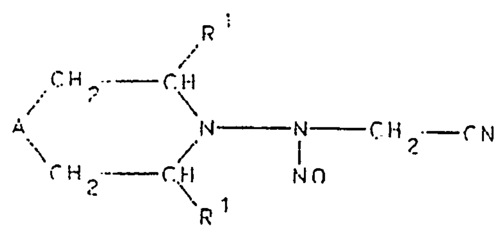

(I)

und ihre pharmakologisch annehmbaren Säureadditionssalze, worin

A den Rest —$CH_2$—, —O—, —S($O_n$)—, —N($R^2$)— oder eine direkte Bindung,

$R^1$, $R^2$ Alkyl mit 1 bis 4 C-Atomen oder Penylalkyl mit 1 bis 4 C-Atomen im Alkylrest,

n die Zahl 0, 1 oder 2,

bedeuten, als pharmakologische Wirkstoffe zur Herstellung pharmazeutischer Präparate, die der Bekämpfung und Vorbeugung cardiovaskulärer Erkrankungen dienen.

## Claims

### Claims for the following Contracting States : AT, BE, CH, DE, FR, GB, IT, LI, NL and SE

1. N-Substituted-N-nitrosoaminoacetonitriles of the general formula I

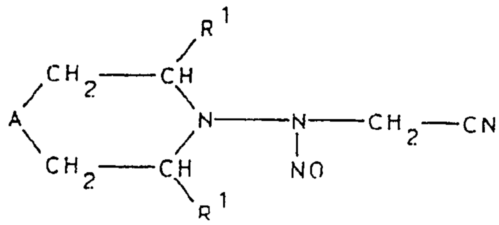

(I)

and their pharmacologically acceptable acid addition salts, wherein

A denotes the radical $—CH_2—$, $—O—$, $—S(O_n)—$, $—N(R^2)—$ or a direct bond,

$R^1$ and $R^2$ denote alkyl having 1 to 4 C atoms or phenylalkyl having 1 to 4 C atoms in the alkyl radical,

n denotes the number 0, 1 or 2.

2. N-Substituted N-Nitrosoaminoacetonitriles according to Claim 1, characterized in that $R^1$ denotes methyl.

3. N-Substituted N-nitrosoaminoacetonitriles according to Claim 1 and/or 2, characterized in that A denotes $—CH_2—$, a direct bond, $—S(O_2)—$ or $—O—$.

4. N-(2,6-Dimethylpiperidin-1-yl)-N-Nitrosoaminoacetonitrile.

5. Process for the preparation of the compounds of the formula I indicated in one or more of Claims 1 to 3 or one of the compounds indicated in Claim 4, characterized in that

a) a compound of the general formula II

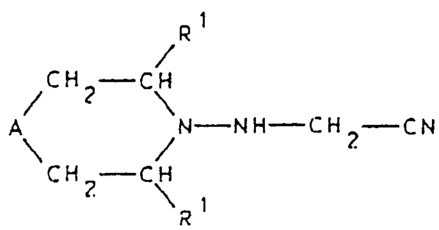

(II)

wherein A and $R^1$ have the meanings mentioned in Claim 1, is nitrosylated, or in that

b) an acid addition salt of a substituted 3-aminosydnone imine of the formula III

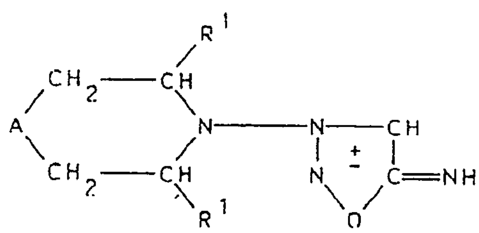

(III)

wherein A und $R^1$ have the meanings mentioned in Claim 1, is reacted with a base and the compound obtained in the case in which A denotes $—N(R^2)—$, is converted, if appropriate, into a pharmacologically acceptable acid addition salt.

6. N-Substituted-N-nitrosoaminoacetonitriles of one or more of Claims 1 to 4 and/or their possible pharmacologically acceptable acid addition salts as pharmacological active compounds for use in the control and phrophylaxis of cardiovascular disorders.

**Claims for the following Contracting States : ES, GR**

1. Process for preparing N-substituted-N-nitrosoaminoacetonitriles of the general formula I

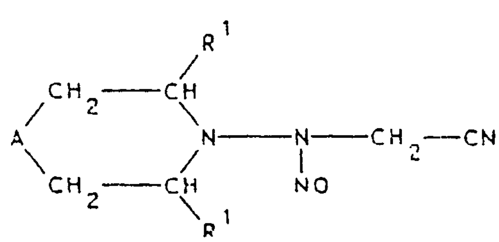

(I)

and their pharmacologically acceptable acid addition salts, wherein

A denotes the radical $—CH_2—$, $—O—$, $—S(O_n)—$, $—N(R^2)—$ or a direct bond,

$R^1$ and $R^2$ denote alkyl having 1 to 4 C atoms or phenylalkyl having 1 to 4 C atoms in the alkyl radical,

n denotes the number 0, 1 or 2, characterized in that

a) a compound of the general formula II

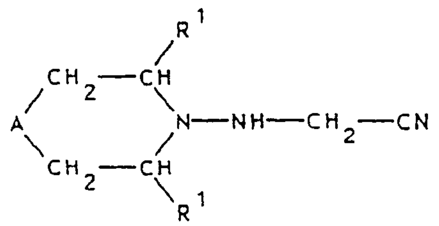

(II)

wherein A and $R^1$ have the meanings mentioned is nitrosylated, or in that

b) an acid addition salt of a substituted 3-aminosydnone imine of the formula III

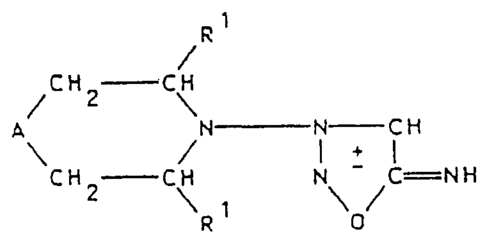

(III)

wherein A und $R^1$ have the meanings mentioned is reacted with a base and the compound obtained in the case in which A denotes $—N(R^2)—$, is converted, if appropriate, into a pharmacologically acceptable acid addition salt.

2. Process according to Claim 1, characterized in that a compound of the formula II or III wherein $R^1$ denotes methyl is used.

3. Process according to Claim 1 and /or 2, characterized in that a compound of the formula II and/or III wherein A denotes $—CH_2—$, $—S(O_2)—$, $—O—$ or a direct bond is used.

4. Process according to one or more of Claims 1 to 3, characterized in that a compound of the formula II or III wherein A denotes $—CH_2—$ and $R^1$ denotes methyl is used.

5. Use of N-substituted-N-nitrosoaminoacetonitriles of the general formula I

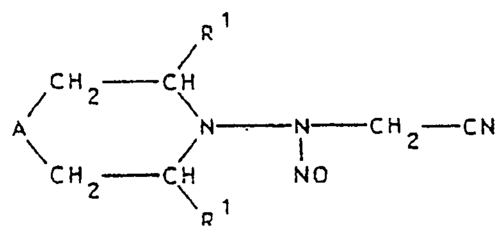

(I)

and their pharmacologically acceptable acid addition salts, wherein

A denotes the radical $—CH_2—$, $—O—$, $—S(O_n)—$, $—N(R^2)—$ or a direct bond,

$R^1$ and $R^2$ denote alkyl having 1 to 4 C atoms or phenylalkyl having 1 to 4 C atoms in the alkyl radical,

n denotes the number 0, 1 or 2,

as pharmacological active compound for preparing pharmaceutical preparations for use in the control and prophylaxis of cardiovascular disorders.

## Revendications

**Revendications pour les Etats contractants suivants : AT, BE, CH, DE, FR, GB, IT, LI, NL, SE**

1. N-Nitroso-aminoacétonitriles substitués à l'azote, qui répondent à la formule générale I :

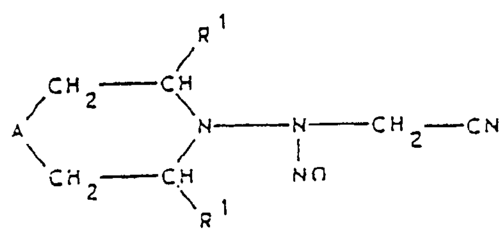

(I)

dans laquelle :

A représente un radical $-CH_2-$, $-O-$, $-S(O_n)-$, $-N(R^2)-$ ou une liaison directe,

$R^1$ et $R^2$ représentent chacun un alkyle contenant de 1 à 4 atomes de carbone ou un phénylalkyle dont l'alkyle contient de 1 à 4 atomes de carbone, et

n désigne un nombre égal à 0, à 1 ou à 2, ainsi que les sels d'addition acceptables du point de vue pharmacologique qu'ils forment avec des acides.

2. N-Nitroso-aminoacétonitriles substitués à l'azote selon la revendication 1, caractérisés en ce que $R^1$ représente un radical méthyle.

3. N-Nitroso-aminoacétonitriles substitués à l'azote selon l'une des revendications 1 et 2, caractérisés en ce que A représente $-CH_2-$, une liaison directe, $-S(O_2)-$ ou $-O-$.

4. N-(Diméthyl-2,6 pipéridyl-1)-N-nitroso-aminoacétonitrile.

5. Procédé pour préparer les composés de formule I indiqués dans l'une quelconque des revendications 1 à 3 ou le composé indiqué à la revendication 4, procédé caractérisé en ce que :

a) on nitrose un composé répondant à la formule générale II :

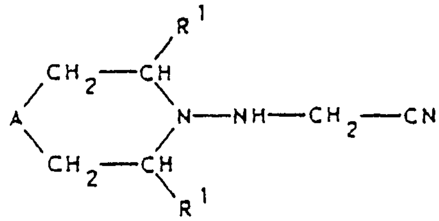

(II)

dans laquelle A et $R^1$ ont les significations qui leur ont été données à la revendication 1, ou

b) on fait réagir avec une base un sel d'addition d'acide d'une amino-3 sydnonimine substituée répondant à la formule III :

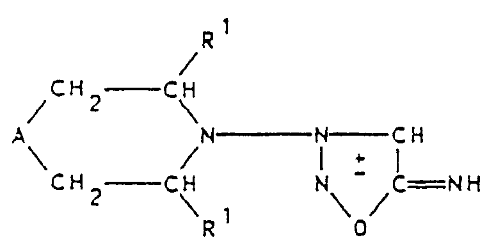

(III)

dans laquelle A et $R^1$ ont les significations qui leur ont été données à la revendication 1, et on transforme éventuellement le composé obtenu, dans le cas où A représente un radical $-N(R^2)-$, en un sel d'addition d'acide acceptable du point de vue pharmacologique.

6. N-Nitroso-amino-acétonitriles substitués à l'azote selon l'une quelconque des revendications 1 à 4 et/ou, s'ils existent, leurs sels d'addition d'acides acceptables du point de vue pharmacologique, en tant que substances actives pharmacologiques pour l'emploi dans le traitement et la prévention de maladies cardiovascu-

laires.

**Revendications pour les Etats contractants suivants : ES, GR**

1. Procédé pour préparer des N-nitrosoaminoacétonitriles substitués à l'azote, qui répondent à la formule générale I :

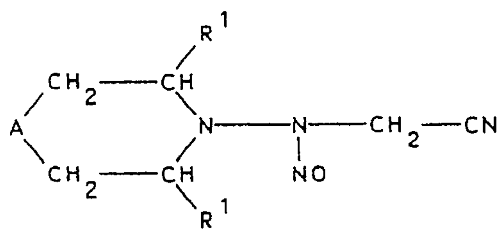

(I)

dans laquelle :

A représente un radical —$CH_2$—, —O—, —$S(O_n)$—, —$N(R^2)$— ou une liaison directe,

$R^1$ et $R^2$ représentent chacun un alkyle contenant de 1 à 4 atomes de carbone ou un phénylalkyle dont l'alkyle contient de 1 à 4 atomes de carbone, et

n désigne un nombre égal à 0, 1 ou à 2,

ainsi que les sels d'addition acceptables du point de vue pharmacologique qu'ils forment avec des acides,

a) on nitrose un composé répondant à la formule générale II :

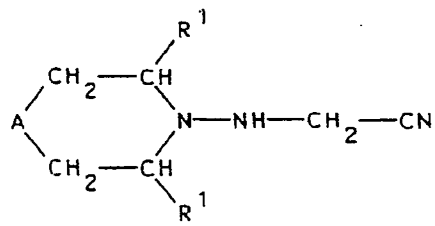

(II)

dans laquelle A et $R^1$ ont les significations indiquées ou

b) on fait réagir avec une base un sel d'addition d'acide d'une amino-3 sydnonimine substituée répondant à la formule III :

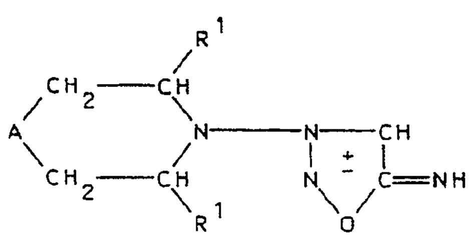

(III)

dans laquelle A et $R^1$ ont les significations indiquées et on transforme éventuellement le composé obtenu, dans le cas où A représente un radical —$N(R^2)$—, en un sel d'addition d'acide acceptable du point de vue pharmacologique.

2. Procédé selon la revendication 1, caractérisé en ce qu'on utilise un composé de formule II ou III dans laquelle $R^1$ représente un radical méthyle.

3. Procédé selon l'une des revendications 1 et 2, caractérisé en ce qu'on utilise un composé de formule II et/ou III dans laquelle A représente —$CH_2$—, —$S(O_2)$—, —O— ou une liaison directe.

4. Procédé selon l'une quelconque des revendications 1 à 3, caractérisé en ce qu'on utilise un composé de formule II ou III dans laquelle A représente —$CH_2$— et $R^1$ représente un radical méthyle.

5. Usage des N-nitroso-amino-acétonitriles substitués à l'azote, qui répondent à la formule générale I :

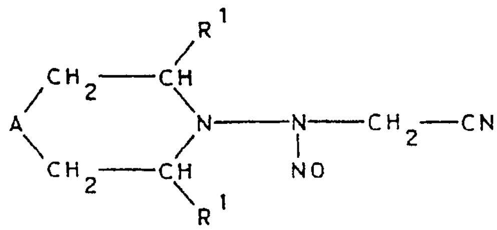

(I)

dans laquelle :

A représente un radical $—CH_2—$, —O—, $—S(O_n)—$, $—N(R^2)—$ ou une liaison directe,

$R^1$ et $R^2$ représentent chacun un alkyle contenant de 1 à 4 atomes de carbone ou un phénylalkyle dont l'alkyle contient de 1 à 4 atomes de carbone, et

n désigne un nombre égal à 0, 1 ou à 2,

et leurs sels d'addition d'acides acceptables du point de vue pharmacologique, en tant que substances actives pharmacologiques pour préparer des compositions pharmaceutiques pour l'emploi dans le traitement et la prévention de maladies cardiovasculaires.